# EUROPEAN PATENT APPLICATION

(11) **EP 4 368 135 A1**
(43) Date of publication of application: **15.05.2024**
(21) Application number: 22206792.8
(22) Date of filing: 10.11.2022
(51) Int. Cl.: A61B 18/18

(54) **A MICROWAVE ABLATION PROBE**

(71) Applicant: Endowave Ltd., D02 DK18 Dublin 2 (IE)
(72) Inventor: Ruvio, Giuseppe, Dublin 2, D02 DK18 (IE); Eaton-Evans, Jimmy, Dublin 2, D02 DK18 (IE); Hogan, Simon, Dublin 2, D02 DK18 (IE); Bergin, Declan, Dublin 2, D02 DK18 (IE); Farina, Laura, Dublin 2, D02 DK18 (IE); Walsh, Eoin J., Dublin 2, D02 DK18 (IE); Walsh, Paul, Dublin 2, D02 DK18 (IE)
(74) Representative: Barker Brettell LLP

(57) **Abstract**

A microwave ablation probe (100; 200; 300; 400; 500). The microwave ablation probe comprising a dipole antenna (106; 206; 306; 406; 506) arranged to emit microwave radiation to heat surrounding tissue; and a feed cable arranged to supply electromagnetic energy to the dipole antenna, the feed cable comprising a coaxial cable having an inner conductor (108; 208; 308; 408; 508), an outer conductor (110; 210; 310; 410; 510) and a dielectric (112; 212; 312; 412; 512) therebetween. The dipole antenna (106; 206; 306; 406; 506) comprises a first antenna section (106a; 206a; 306a; 406a; 506a) forming a first pole of the dipole antenna and a second antenna section (106b; 206b; 306b; 406b; 506b) forming a second pole of the dipole antenna. The first antenna section (106a; 206a; 306a; 406a; 506a) comprises one or more antenna elements (116a-i; 216a-i; 316a-i; 416a-h; 516a-h) each formed integrally with the outer conductor (110; 210; 310; 410; 510) and comprising an outwardly extending portion (117a) extending away from a longitudinal axis of the feeding cable and bending back over an insulator (118; 318; 418; 518) to form an overlapping portion (117b) extending proximally along the length of the feeding cable. The first antenna section (106a; 206a; 306a; 406a; 506a) further comprises an antenna insulator (118; 318; 418; 518) between the overlapping portion of each of the one or more antenna elements and the part of the outer conductor which they overlap.

## Description

This application relates to a microwave ablation probe and a method of manufacturing an ablation probe. In particular, this application relates to the antenna of an ablation probe that may be used to generate heat within tissue to destroy tissue growths.

Thermal ablation can be used to destroy tissue growths within the body which can be malignant. Current ablation systems use applicators that deliver Radio Frequency (RF) energy or more specifically microwave energy to the tissue surrounding an applicator tip. This causes localised heating and destruction of the malignant cells.

Known ablation probes comprise an active tip (or applicator) generally made of ceramic material which is coupled to a coaxial feed cable arranged to supply electromagnetic energy to the active tip. The inner conductor of the coaxial feed cable extends through the ceramic material and forms a monopole antenna with the ceramic material acting as a resonator.

When applying microwaves to heat surrounding tissue it is advantageous to produce a spherical ablation zone around the applicator in which tissue is ablated. This helps to ensure that ablation can be directed more accurately to the relevant tissue. In order to produce a spherical ablation zone a dipole antenna may be used. Such an antenna may have two antenna sections which emit radiation from a central point between them.

The design of a dipole antenna for an ablation probe is challenging because of the limited available space for components in a device small and flexible enough to insert into the body and the need to provide a stable spherical ablation zone within those design constraints. The present application provides an ablation probe having an antenna aimed at overcoming one or more of these challenges.

An aspect of the present disclosure provides a microwave ablation probe, comprising any one or more of the following features:
a dipole antenna arranged to emit microwave radiation to heat surrounding tissue; and
a feed cable arranged to supply electromagnetic energy to the dipole antenna, the feed cable comprising a coaxial cable having an inner conductor, an outer conductor and a dielectric therebetween, wherein:
   the dipole antenna comprises a first antenna section forming a first pole of the dipole antenna and a second antenna section forming a second pole of the dipole antenna;
   the first antenna section comprises one or more antenna elements each formed integrally with the outer conductor and comprising an outwardly extending portion extending away from a longitudinal axis of the feeding cable and an overlapping portion extending proximally along the length of the feeding cable; and
   the first antenna section further comprises an antenna insulator between the overlapping portion of each of the one or more antenna elements and the part of the outer conductor which they overlap.

By forming the antenna elements integrally with the outer conductor in this way one or more of the following advantages may be provided:
- Electrical continuity between the antenna elements and outer conductor may be improved. This may improve the electrical performance of the ablation probe.
- Less components are required to be manufactured and assembled in order to produce the antenna.
- It is easier to expose the cable dielectric without damaging it. This again may improve electrical performance and aid manufacture.
- Using part of the outer conductor to form the antenna elements helps to avoid the need to machine electrically conductive antenna components which simplifies manufacturing processes.
- The flexibility of the ablation probe may be improved at the antenna, which may be particularly useful for delivery though an intraluminal delivery device.
- A low profile may also be provided, which is advantageous for insertion into a working channel or into tissue.
- A number of clinical advantages may also be achieved. For example, greater flexibility may allow difficult to reach anatomy to be accessed, e.g. the periphery of the lung. The low profile may reduce tissue damage and the improved electrical performance may allow more stable and/or more spherical ablation zones to the created to target issue accurately.

The outer conductor may be a braided outer conductor. The braided outer conductor may comprise a plurality of braided wire strands. Each antenna element may be formed by one or more of the wire strands. The antenna elements may be formed by un-braiding part of the cable and re-using the strands that form the braid so that they are integrally formed with the outer conductor.

The wire strands forming the antenna elements may be overlapped or woven together such that the first antenna section is formed by a re-braided (or at least partly re-braided) section of the outer conductor. The outer conductor may be un-braided and then at least partly re-braided to form the first antenna section.

The outer conductor may comprise a solid conducing sheath or tube of material around the dielectric.

The antenna elements of the first antenna section may each be formed by part of the solid conducting sheath that is shaped to form the respective outwardly extending and overlapping portions of the antenna elements. The solid conducting sheath may be cut to form separate antenna elements integral with the sheath. For example, the solid sheath can be cut to form strips of material that can be shaped into the antenna elements. Cut-outs of material can be removed from between the strips so that they can be more easily bent into shape.

The first antenna section may comprise at least two antenna elements. The antenna elements may be spaced (preferably evenly spaced) apart circumferentially around the feeding cable.

The overlapping portion or portions of the antenna elements may extend along a helical path along the longitudinal axis of the ablation probe. For example, they may extend helically around the outer surface of the antenna insulator.

The second antenna section may comprise:
a length of the inner conductor extending distally past the antenna elements of the first antenna section along the length of the ablation probe; and
a dielectric resonator into which the inner conductor extends.

A proximal end of the dielectric resonator may be spaced apart from the antenna elements of the first antenna section along the length of the inner conductor to form a flexible hinge region therebetween. The hinge may allow preferential bending of the ablation probe at its position within the length of the probe.

The microwave ablation probe may further comprise a catheter tube in which (at least part of) the coaxial cable and the antenna are located. The catheter tube may therefore form part of the feed cable.

The region of the catheter tube which surrounds or is adjacent the antenna may be formed from a polymer material or materials and is free of electrically conducting components. This may help it to be transparent to microwave propagation and so not affect the ablation in surrounding tissue.

The entire length of the catheter tube may be formed from a polymer material or materials and is free of electrically conducting components. This may avoid the need to join sections of tube made from different materials.

The polymer material(s) may include any one or more of:
i) a polymer or polymers from the Fluoropolymer or Fluoroelastomer families, for example fluorinated ethylene propylene (FEP) and/or polytetrafluoroethylene (PTFE); and/or
ii) a polymer (e.g. PEEK) reinforced laminated tube.

These materials may allow the desired level of flexibility and durability of the catheter tube where it is stressed by high temperature, pressure, insertion and withdrawal forces when in use, while also being transparent to microwave radiation.

The dielectric resonator may comprise a plurality of (e.g. radially extending) resonator protrusions. The resonator protrusions may extend from an outer surface of the dielectric resonator.

The catheter tube may be spaced apart from the outer surface of the dielectric resonator by the plurality of resonator protrusions.

The resonator protrusions may be arranged to locate the catheter tube concentrically with the dielectric resonator.

The first antenna section may comprise a plurality of antenna elements. The antenna insulator may comprise a plurality of (e.g. radially extending) insulator protrusions (e.g. insulator fins) extending between the antenna elements.

The insulator protrusions may be arranged to locate the catheter tube concentrically with the antenna insulator.

The microwave ablation probe may further comprise one or more coolant channels between the resonator protrusions and/or the insulator protrusions.

The antenna insulator may form a flexible region of the ablation probe.

The antenna insulator may comprise: a) a solid flexible material; or b) a plurality of rigid beads.

The antenna insulator may allow flow of coolant therethrough. The antenna insulator may comprise one or more coolant channels extending through it to allow coolant to pass through the antenna.

The antenna insulator may comprise one or more airgaps (e.g. cavities filled with air) and may be formed from a ribbed polymer comprising one or more air gaps. The airgaps may help to improve the flexibility of the insulator and may help to improve the electrical insulation provided by the insulator.

The ablation probe may comprise a multi-lumen extrusion or tube (e.g. the feed cable may comprise a multi-lumen extrusion). The antenna insulator may be formed by part of the multi-lumen extrusion.

The multi-lumen extrusion may be a tube having a plurality of lumens which is formed from a single integral extrusion of material (e.g. a polymeric material). By forming the antenna insulator from part of a multi-lumen extrusion the number of components required may be reduced.

The multi-lumen extrusion may comprise a central inner lumen defined by an inner extrusion wall. The coaxial cable may be arranged within the central inner lumen. Part of the inner extrusion wall may form the antenna insulator.

The multi-lumen extrusion may further comprise one of more outer lumens. The outer lumens may be defined by the outer wall of the inner extrusion wall, an outer extrusion wall and optionally one or more radially extending extrusion walls. The overlapping portions of the antenna members may be located within the outer lumens. The outer extrusion wall may form the catheter tube described in any of the statements above.

One or more of the outer lumens may be arranged to carry a flow of coolant along the length of the ablation probe. A first of the outer lumens may be arranged to carry a flow of coolant in a distal direction along the ablation probe and a second of the lumens may be arranged to carry a flow of coolant in a proximal direction along the length of the ablation probe. This may allow a coolant circuit to be formed to cool the antenna.

The overlapping portions of the one or more antenna elements may be embedded into the antenna insulator.

The overlapping portions of the one or more antenna elements may be crimped around the insulator.

The first antenna section may further comprise a heat-shrink sleeve arranged to surround and retain the overlapping portions of the one or more antenna elements around the antenna insulator.

The first antenna section may further comprise a wire (e.g. a polymer or metallic wire) arranged to surround and retain the overlapping portions of the one or more antenna elements around the antenna insulator.

The first antenna section may further comprise one or more radio-opaque marker bands arranged to surround and retain the overlapping portions of the one or more antenna elements around the antenna insulator.

The first antenna section may further comprise one or more electrically conductive bands arranged to surround and retain the overlapping portions of the one or more antenna elements around the antenna insulator. The bands may be formed from strips of conducting material extending around the antenna insulator and overlapping portions to hold them in position. The electrically conductive bands may be formed by a ring or rings of solder around the antenna elements.

The antenna insulator of the first antenna section may comprise one more recesses in its surface each arranged to receive a respective one of the antenna elements. This may help to retain the antenna elements in position.

The first antenna section may comprise a glue arranged to retain the overlapping portions of the one or more antenna elements around the antenna insulator.

According to a second aspect, there is provided a method of manufacturing an ablation probe, the ablation probe comprising:
a dipole antenna arranged to emit microwave radiation to heat surrounding tissue, the dipole antenna comprising a first antenna section forming a first pole of the dipole antenna and a second antenna section forming a second pole of the dipole antenna; and
a feed cable comprising a coaxial cable arranged to supply electromagnetic energy to the dipole antenna, the method comprising:
   providing a coaxial cable comprising an inner conductor, an outer conductor and a dielectric therebetween;
   forming the first antenna section by shaping a part of outer conductor such that it forms one or more antenna elements each comprising a outwardly extending portion of the outer conductor extending away from a longitudinal axis of the feeding cable and an overlapping portion of the outer conductor extending proximally along the length of coaxial cable; and
   providing an insulator between the overlapping portions of the one or more antenna elements and the part of the outer conductor which they overlap.

The outer conductor may comprise a plurality of braided conducting wire strands. Forming the antenna elements of the first antenna section may comprise:
un-braiding the braided strands of part of the outer conductor of the coaxial cable; and
shaping the un-braided stands to form the one or more antenna elements of the first antenna section.

The outer conductor may comprise a solid conducting sheath or tube of material around the dielectric. Forming the antenna elements of the first antenna section may comprise:
cutting the sheath or tube to form one or more strips integrally formed with the remainder of the outer conductor material from which they are cut; and
shaping each of the one or more strips to form the one or more antenna elements of the first antenna section.

The cutting of the sheath may comprise laser cutting the sheath.

The ablation probe manufactured by the method of the second aspect may be the ablation probe of the first aspect.

Any features described above in connection with one aspect or statement may be used, unless where mutually exclusive, in combination with any other aspect or statement.

As used herein, a range "from value X to value Y" or "between value X and value Y", or the like, denotes an inclusive range; including the bounding values of X and Y. Angles are given in degrees unless otherwise stated.

Embodiments of the invention will now be described, by way of example only, with reference to the accompanying drawings, in which:
**Figure 1** shows a schematic view of an ablation probe according to an embodiment;
**Figure 2** shows cross-sectional view of the antenna of the ablation probe shown in Figure 1;
**Figure 3a** shows cross-section view through the antenna of Figure 2 through the plane marked A-A;
**Figure 3b** shows a side view of the antenna of Figure 3a without the antenna insulator, including a close up of the outer conductor;
**Figure 4a** shows cross-section view corresponding to that of Figure 3a illustrating an alternative embodiment of the outer conductor;
**Figure 4b** shows a side view corresponding to that of Figure 3b illustrating the alternative embodiment of the outer conductor shown in Figure 4a;
**Figure 5** shows a side view of an alterative embodiment of the antenna shown in Figure 2 having a solid outer conductor rather than a braided outer conductor;
**Figures 6a and 6b** illustrate how antenna elements are formed by cutting the outer conductor of a coaxial cable;
**Figures 7a and 7b** illustrate the relative size and spacing of the antenna elements of the antenna;
**Figure 8** shows a cross-sectional view of the antenna of an ablation probe according to another embodiment having a hinge region;
**Figure 9** shows a cross-sectional view of the applicator section of an ablation probe according to another embodiment;
**Figures 10 and 11** show cross-section views through the applicator section shown in Figure 9 through the planes marked C-C and D-D;
**Figure 12** shows a perspective view of parts of the antenna of the ablation probe shown in Figure 9;
**Figure 13** shows a side view of the antenna of an ablation probe according to another embodiment;
**Figure 14** shows a cross-section through the antenna shown in Figure 13 through the plane marked E-E;
**Figure 15** shows an alternative view to that shown in Figure 13 but without the heat-shrink sleeve;
**Figures 16a** **to c** show a multi-lumen tube arranged to form the antenna insulator of the antenna of an ablation probe according to other embodiments; and
**Figure 17** shows a method of manufacturing an ablation probe.

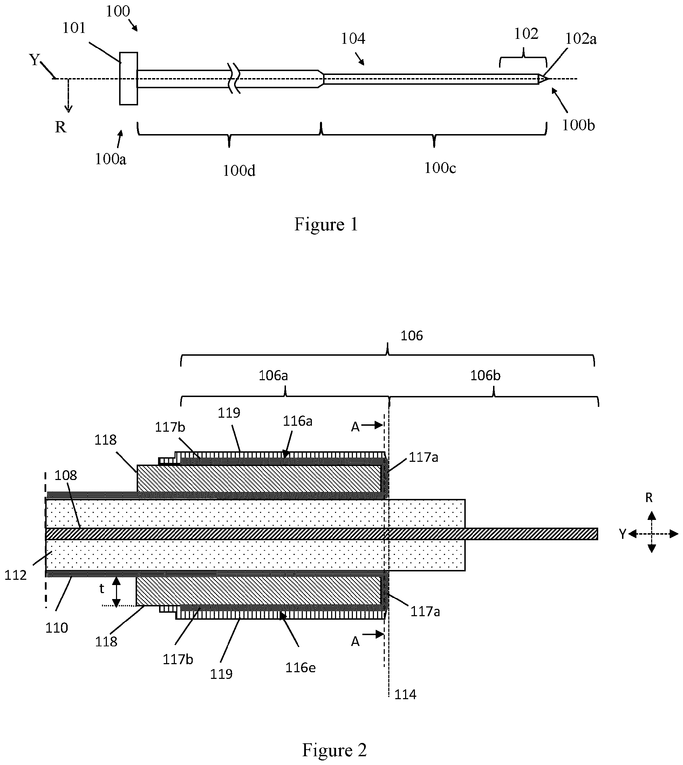

An ablation probe 100 according to an embodiment is shown schematically in Figure 1. The ablation probe 100 of the present disclosure may be suitable for insertion into the body to reach a desired treatment site, such as a malignant tissue growth. In order to reach a desired treatment site, the ablation probe may be suitable for insertion through the working channel of an internal anatomy access device. By internal anatomy access device we mean any device which may be placed within the anatomy of a patient, the device having a working channel for insertion of instruments to a desired location within the body. The internal anatomy device may be an intraluminal delivery device arranged to be delivered along an anatomical lumen of the patient (e.g. the trachea and the pathways of the bronchi in the lungs or the oesophagus). The ablation probe 100 may, for example, be used endoscopically or using an ENB (electromagnetic navigation bronchoscopy) system in order to reach a variety of disease locations within the body. The ablation probe may therefore have an overall flexibility such that it can be inserted through the working channel of an endoscope or similar device. In other embodiments, the ablation probe may be used with other types of intraluminal delivery device such as specific types of endoscope (e.g. a bronchoscope) or a navigation system such as a lung navigation system (e.g. an ENB system). In other examples, the ablation probe 100 may also be used percutaneously, or using any other suitable technique, e.g. inserted through an existing aperture of the body. For percutaneous use, the ablation probe may be generally rigid so that it can be inserted.

The ablation probe extends between a proximal end 100a and a distal end 100b. The terms "distal" and "proximal" are taken relative to the user operating the ablation probe and the treatment site when the ablation probe is positioned for use - the distal end 100b of the ablation probe 100 is that closest to the treatment site and the proximal end 100a is that closest to the user. The ablation probe 100 has a longitudinal axis Y that extends the length of the device between the distal and proximal ends. A radial direction R is defined as a direction normal to the longitudinal axis Y as shown in Figure 1. A reference to being along the "length" of the ablation probe or other component herein refers to the length in a direction along or parallel to the longitudinal axis Y.

A handle 101 is provided at the proximal end of the ablation probe 100 so that it can be manipulated and positioned by the user. The distal end 100b may be fed through the working channel of an endoscope or similar device to reach a target ablation site.

The ablation probe 100 generally comprises an applicator section 102 and a feeding or feed cable 104. The terms "feeding cable" and "feed cable" are used interchangeably herein. The applicator 102 comprises an antenna 106 and is arranged to emit radiation to heat surrounding tissue when the ablation probe is in use. The emitted radiation may be used to cause localised heating and destruction of malignant cells around or near to the applicator 102. The applicator 102 may be arranged to apply any suitable form of radiation to surrounding tissue such that the desired heating is caused. The applicator 102 may, for example, be arranged to emit microwave or RF radiation, or may emit any other suitable radiation to cause heating. The applicator 102 is arranged at or near a distal end of the ablation probe 100 so that it can be positioned in a desired position relative to the tissue to be treated.

The feed cable 104 is arranged to supply electromagnetic energy to the applicator 102. Only part of the length of the feeding cable is shown in the Figures. The feeding cable 104 may be an elongate member suitable for supplying electromagnetic energy to the applicator (e.g. a conductor), but may include additional components (e.g. coolant conduits). The feeding cable 104 may run along at least part or all of the length of the ablation probe 100 to deliver a supply of energy to the applicator 102. In the described embodiment, the applicator is located at the distal end of the feeding cable 104 and a proximal end of the feeding cable 104 is coupled to a generator (not shown in the Figures) suitable for generating the desired frequency signal to supply energy to the applicator 102. In the presently described embodiment, the feeding cable 104 comprises a coaxial cable as will be described in more detail later. An additional length of coaxial cable not shown in the figures may be used to connect the distal end of the feed cable (e.g. at the handle 101) to the signal generator. Similar additional connections may be provided for a coolant circuit which are also not shown in the Figures.

In the present embodiment, an applicator tip 102a is attached at the distal end of the applicator 102. The tip 102a forms a pointed distal tip of the ablation probe 100 that is suitable for piercing tissue in use. In some embodiments, the tip may be a separate component. In other embodiments, a separate tip component may not be provided, such that the tip is formed integrally with the applicator 102. It may therefore be integral with, or connected to, a dielectric resonator of the applicator. In yet other embodiments, the ablation probe may have an atraumatic distal tip, rather than a pointed one.

Referring again to Figure 1, the ablation probe 100 generally comprises two portions: a needle portion 100c and a catheter portion 100d. The needle portion 100c may be arranged at the distal end of the ablation probe 100 and is adapted to be inserted into tissue during use to reach the desired ablation location. The catheter portion 100d may be provided at the proximal end of the ablation probe 100 and is arranged to supply electromagnetic energy to the needle portion (and optionally a flow of coolant to and from the needle portion 100. The catheter portion 100d may have an extended length and flexibility for endoscopic use. In other embodiments, a shorter, more rigid catheter portion 134 may be provided for percutaneous use.

In some embodiments, the needle portion 100c may form a small part of the overall length of the ablation probe. For example, the needle portion may be 5 mm to 2000 mm in length, and preferably may be around 70 mm in length. The length of the needle portion 100c may be chosen according to the anatomy to be accessed. For example, the needle portion may be approximately between 10 and 100 mm long for delivery of therapy to organs including the pancreas, or lung, or longer (for example 100-400 mm in length) for delivery of therapy percutaneously. A longer length of needle portion may be more suitable for accessing parts of the lung, for example. The catheter portion may be around 1000 mm to 2000 mm in length, and preferably around 1400 mm in length. The length of the catheter portion may be chosen according to the position of the ablation site which must be reached.

In other embodiments, the needle portion 100c of the ablation probe may form a greater proportion of the length of the ablation probe. In some embodiments, the entire length of the ablation probe may be formed by the needle portion 100c (i.e. such that a separately defined catheter portion is absent). For example, if the ablation probe is to be used percutaneously the catheter portion 100d may be shorter than for endoscopic use, or may not be required.

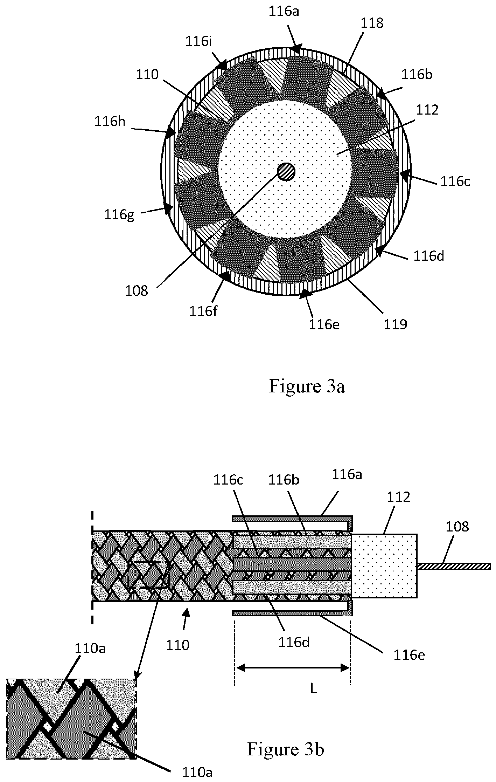

Referring now to Figure 2, a schematic cross-sectional view (in a plane through the longitudinal axis Y) of the antenna 106 of the ablation probe 100 is shown. Figure 3a shows a cross section through the plane marked A-A in Figure 2. Figures 2 and 3a show the parts of the antenna 106 and feed cable 104 in isolation to aid explanation; other components may be present even though they are not shown. The antenna 106 is a dipole antenna arranged to emit microwave radiation to heat surrounding tissue as described above. The dipole antenna comprises a first antenna section 106a forming a first pole of the dipole antenna 106, and a second antenna section 106b forming a second pole of the dipole antenna. The antenna 106 is fed with a microwave signal from a microwave generator by a coaxial cable provided in the feed cable 104. The coaxial cable comprises an inner conductor 108, an outer conductor 110, and a dielectric 112. The dielectric is located between the inner conductor 108 and the outer conductor 110. The antenna 106 is arranged to produce a spherical (or approximately spherical) ablation zone centred on the feed point 114 of the antenna 106. The feed point 114 is the point along the length of the ablation probe at which the inner conductor 108 is first exposed outside of the outer conductor (i.e. the point just past the point at which the outer conductor extends no further along the longitudinal length of the ablation probe). The first antenna section 106a is electrically connected to the outer conductor 110 of the coaxial cable, and the second antenna section 106b electrically connected to the inner conductor 108 to form a dipole antenna.

The first antenna section 106a comprises a plurality of antenna elements (or antenna members) 116a-i which are each formed integrally with the outer conductor 110. Two such antenna elements 116a, 116e can be seen in the cross section of Figure 2. In the present embodiment, the first antenna section 106a comprises nine antenna elements as can be seen in Figure 3a. Other numbers of antenna elements may be provided in other embodiments. In some embodiments, the first antenna section 106a may comprise one only antenna element 116a, but preferably it comprises at least two as will be described later. Each of the antenna elements 116a-i comprises an outwardly extending portion 117a that extends in a direction outwards away from the longitudinal axis of the ablation probe e.g. in a direction having a component in a radial direction R away from the longitudinal axis Y of the ablation probe (which corresponds to the longitudinal axis of the coaxial cable). The outwardly extending portions may therefore be referred to as radially extending portions. Each antenna element 116a-i further comprises an overlapping portion 117b extending proximally along the length of the coaxial cable (e.g. in a direction parallel to the longitudinal axis Y). The radial portion and the overlapping portion are formed integrally with the rest of the outer conductor i.e. they are a continuous piece of material with no joints (such as solder joints or welds) between them. The outwardly extending and overlapping portions are formed by shaping (e.g. bending) a part of the outer conductor so that it extends away from and back along the coaxial cable so that part of the dielectric is exposed as shown in Figure 2. Although the outwardly extending portions 117a are shown in Figure 2 to extend in a direction perpendicular to the longitudinal axis Y they may extend at any angle so long as they are bent outwards to extend away from the longitudinal axis of the coaxial cable (e.g. in a direction having a component in the radial direction R). Similarly the overlapping portions may have any shape such that they extend proximally back along the coaxial cable.

The first antenna section 106a further comprises an antenna insulator 118 arranged to separate or space apart the overlapping portion 117b of each antenna element 116a-i from the part of the outer conductor 110 which it overlaps. The antenna elements 116a-i may lie on the outside surface of the insular material as shown in the figures. In other embodiments, they may be embedded into the insulator material, or located in recesses in its surface as will be described later. The insulator 118 is arranged to electrically insulate the overlapping portions 117b from the adjacent part of the outer conductor 110. In the present embodiment, the insulator 118 comprises an annular component which fits around the outer conductor 110. It therefore forms an insulating layer between the outer conductor 110 and overlapping portions 117a of the antenna elements 116a-i.

The insulator 118 generally forms a flexible structure such that it may bend or flex during use of the ablation probe. This allows the ablation probe to extend through the anatomy of the patient along a tortuous path when used with an intraluminal delivery device. In the present embodiment, the insulator is formed from a (solid) flexible insulator material. In other embodiments, the insulator may comprise a plurality of rigid beads or pellets. Although each bead in such an embodiment is rigid, an overall flexible structure is produced.

By forming the first antenna section by shaping parts of the outer conductor 110 to form the antenna elements 116a-i a number of advantages may be achieved:
- Electrical continuity may be provided between the antenna sections and the rest of the outer conductor compared to if separate components were attached to the outer conductor. This may improve the electrical performance of the antenna (e.g. may minimise losses and help produce a spherical ablation zone).
- By forming the antenna elements as described above it is easier to expose the dielectric layer of the coaxial cable without damaging it. This again may help to maintain electrical performance of the cable.
- Using part of the outer conductor helps avoid machining separate electrically conductive components which would need to be soldered to the cable.
- Less components are required to make the antenna which reduces the overall number of components and processes required to manufacture the antenna.
- The antenna elements allow the antenna to be manufactured such that it has a low cross-sectional profile. Such a low profile is advantageous for allowing the ablation probe to be inserted into the body.
- Forming the antenna elements as described above may help to improve the flexibility of the ablation probe compared to using a more rigid structure for the antenna formed by using additional components.

Forming the first antenna section as described herein may have a number of clinical advantages when the ablation probe is used. For example, its flexibility may be improved so that the applicator can be navigated into the body to reach difficult to access locations e.g. the periphery of the lung. It also helps to keep an overall low cross section profile of the device to allow it to be inserted into a working channel and/or into tissue. It also helps to deliver a stable spherical ablation which may help to target ablation accurately.

In some embodiments, the antenna insulator may be formed from a ribbed polymer comprising one or more air gaps. The airgaps may help to improve the flexibility of the insulator, and may help to improve the electrical insulation provided by the insulator (e.g. by making use of the electrical insulation properties of air).

In some embodiments, the antenna insulator may be arranged to allow a flow of coolant to pass through it (and therefore through the first antenna section). This may be done by providing coolant channels through the body of the antenna insulator material or allowing coolant to flow between beads forming the insulator.

In the presently described embodiment, the outer conductor 110 of the coaxial cable is a braided outer conductor. The braided structure of the outer conductor is not shown in Figure 2 to aid clarity, but is visible in Figures 3a and 3b. The antenna is shown without the insulator 118 in Figure 3b so that the outer conductor is more visible. The close-up in Figure 3b shows part of the braided structure of the outer conductor in greater detail. The outer conductor 110 may comprise of a plurality of strands 110a which are woven or braided together to form the outer conductor 110 which extends around and shields the inner conductor 108. In the described embodiment, each of the strands 110a is formed by a single wire element as shown in the figures (two of which are labelled in the close-up of Figure 3b). In this embodiment each of the strands is a flat wire strand. Each of the antenna elements 116a-i is formed by one of the flat wire strands that has been un-braided from the outer conductor and shaped accordingly. The shape, number and size of the strands 110a shown in the figures is to be understood as one example only.

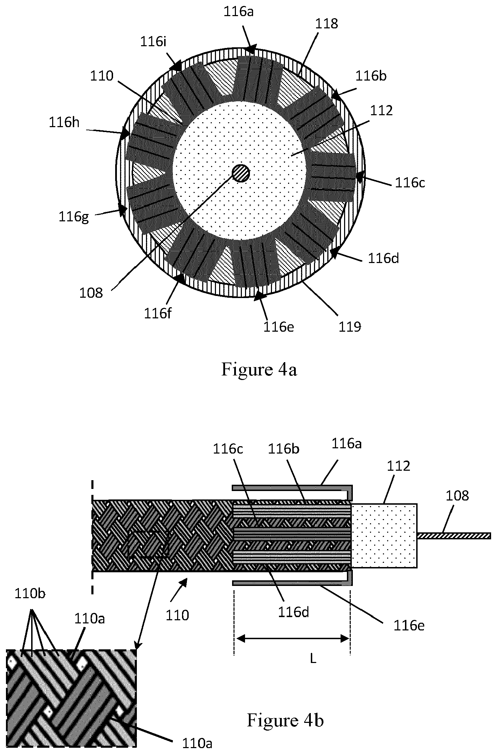

In another embodiment, each of the strands 110a that are braided together to form the outer conductor may themselves each comprise a plurality of separate wire filaments. An example of this is shown in Figures 4a and 4b, which correspond to Figures 3a and 3b but show a different embodiment of the outer conductor. In the example illustrated in Figures 4a and 4b, each of the strands 110a forming the outer conductor comprises four separate wire filaments 110b (four or which are labelled in the close up in Figure 4b) which are grouped together to form each strand making up the braided pattern around the dielectric 212. These wire filaments are also visible in Figure 4a. In other embodiments, the strands 110a may be made up of different numbers of filaments, with only four shown to aid clarity.

As can be seen in Figures 3a to 4b, the antenna elements 116a-i of the first antenna section are formed from an un-braided part of the outer conductor 110. Specifically, in the described embodiments, each antenna element is formed by one of the wire strands of the un-braided part of the outer conductor. This allows the antenna elements to be formed by un-braiding (which includes at least partially un-braiding) the separate strands of the outer conductor such that they are free of each other and shaping them (e.g. bending them) so that they extend away from the outer conductor and proximally back along the cable. This allows the antenna elements to be manufactured integrally with the outer conductor. In other embodiments, the outer conductor can be un-braided and the wire stands forming it used in any other way to create the antenna elements e.g. one or more of the wire strands may be used to form each antenna element, or separate wire filaments of the strands un-braided to form one or more antenna elements. In some embodiments, the wire strands of the outer conductor which form the antenna elements may be re-braided so that they are woven together. For example, a portion of the outer-conductor may be un-braided and then the un-braided stands forming each antenna element re-woven or re-braided into a new braided section of the outer conductor.

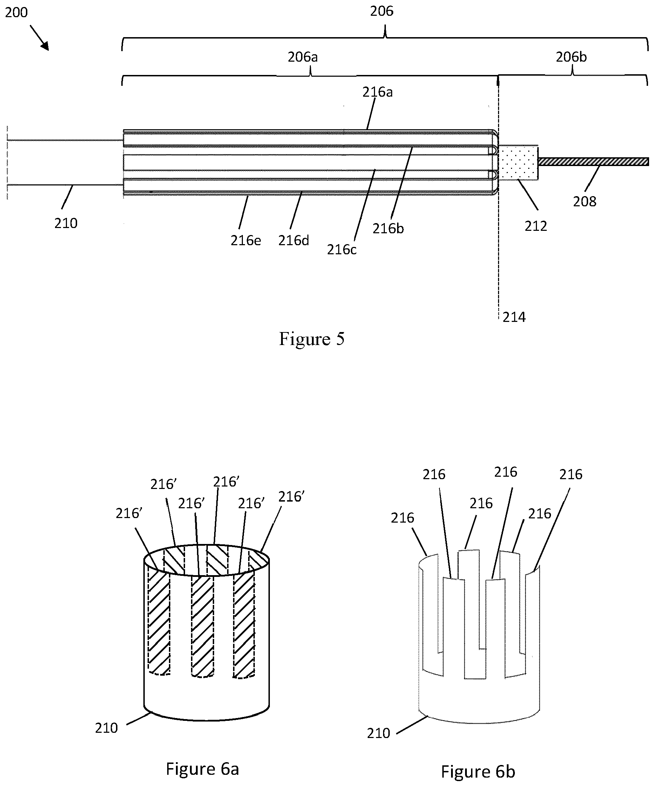

In other embodiments, the outer conductor comprises a solid conducing sheath or tube of material surrounding the dielectric rather than braided wire strands. The outer conductor is therefore a single solid element, rather than made of individual strands. An embodiment of such an antenna 206 is shown in Figure 5. The embodiment of Figure 5 includes similar components (apart from the outer conductor) to that of Figures 1 to 4b, with corresponding reference numbers used accordingly. The second antenna section 206a comprises nine antenna elements 216a-i integrally formed from the outer conductor 210, five of which are visible in Figure 5. The antenna elements 216a-216i are each formed by a section of the outer conductor 210. For example, the outer conductor may be cut to form a series of strips of material. Each strip may then be shaped (e.g. bent) to form the outwardly extending portions and overlapping portions of each antenna element. This embodiment may provide similar advantages to that of the embodiments in Figures 1 to 4b by forming the antenna elements integrally with the outer conductor 210.

An example of antenna elements being formed from a solid sheath outer conductor 210 is illustrated in Figures 6a and 6b. In both of these Figures only part of the outer conductor 210 is shown in isolation as a tubular piece of material. In this example, the outer conductor is cut to form six separate antenna elements 216 labelled in Figure 6b. Figure 6a shows cut-outs 216' being formed in the outer conductor by cutting it along the dashed lines shown in the figure. The shaded cut-out regions 216' are then removed to form gaps or spaces between sections of the outer conductor from which the antenna elements are formed. Figure 6b shows the outer conductor 210 once the shaded cut-outs 216' have been removed. In this example, the cut-outs 216' are slots cut into the end of the outer conductor 210 (e.g. by laser cutting), which when removed leave elongate strips or finger shaped antenna elements 216 extending at the end of the outer conductor 216. These antenna elements 216 may then be shaped to formed the antenna members 216a-i illustrated in Figure 5 (e.g. by cutting an appropriate number of them out of the outer conductor). Figures 6a and 6b show one example of the shape of antenna members which may be formed. In other embodiments, the cut-outs may not be required, with antenna elements being formed by cutting without removing any material. The cut-outs however help the antenna elements be bent into shape.

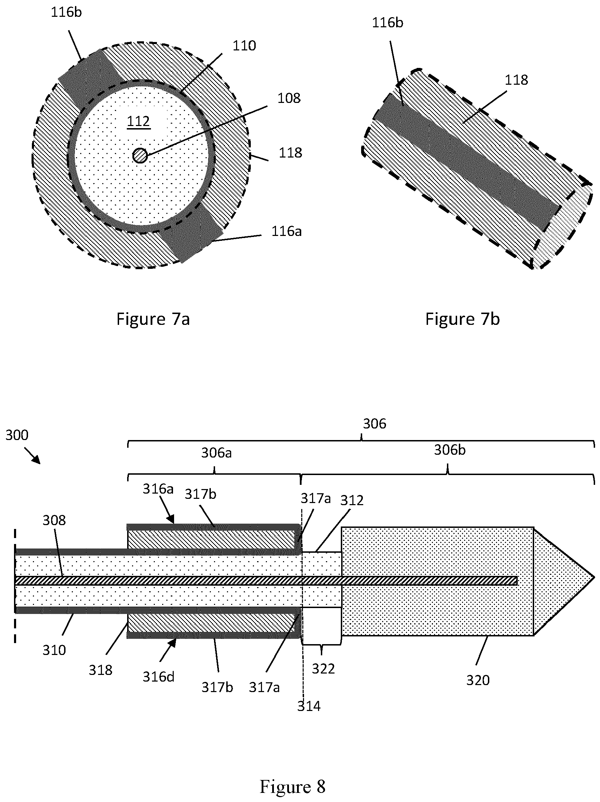

In the embodiments shown in Figures 1 to 5 the first antenna section 106a, 206a comprises nine antenna elements 116a-i, 216a-i. In other embodiments, other numbers of antenna elements may be provided. The inventors have determined that the size and distribution of the antenna elements is important in providing the desired electrical properties of the antenna. More generally, the first antenna section may comprise greater than two antenna elements. This may provide suitable electrical properties of the antenna. Any example of an embodiment having two antenna elements 116a, 116b is shown in Figure 7a, which shows a cross section corresponding to that of Figure 3. In any of the embodiments described herein, each of the antenna elements may extend over an area of 15% (or at least 15%) of the cross-sectional area of the antenna insulator. By cross-sectional area of the antenna insulator we mean the total area over which the antenna insulator extends in a plane normal to the longitudinal axis of the ablation probe i.e. the area between the dashed lines in Figure 7a. In some embodiments, additionally or alternatively, the antenna elements may together extend over at least a total of 20% of the overall longitudinal area of the antenna insulator 118. In other words, the percentage of the outer surface of the antenna insulator covered by the antenna elements is at least 20% of its total area. The longitudinal area of the antenna insulator is defined as the area of the outer surface of the antenna insulator (without the antenna elements) e.g. the area of the cylindrical surface bounded by the dashed lines in Figure 7b, which shows the antenna insulator 118 in isolation being overlapped by one of the antenna elements 116b.

A combination of having at least two antenna elements, each antenna element covering at least 15% each of the cross-sectional area of the insulator and the antenna elements being evenly distributed has been found to provide an electrically efficient contact between the outer conductor of the coaxial cable and the bent-back antenna elements. Any of these features may however be provided independently of each other.

The antenna elements may be evenly distributed around the longitudinal axis of the coaxial cable i.e. they are spaced apart equally circumferentially around the outer conductor as shown in the Figures. This may help to create a uniform ablation zone around the antenna. In other embodiments other arrangements may be provided, including other sizes and spacing of antenna elements.

The thickness of the antenna insulator 118, 218 of any embodiment described herein may be chosen to reduce the risk of an electrical short between the overlapping portions 117b, 217b of the antenna elements 116a-i, 216a-i and the part of the outer conductor which they overlap. In a preferred embodiment, the thickness 't' of the antenna insulator of any embodiment described herein may be in the range of 0.05 mm to 0.5 mm, and may preferably be between 0.1 and 0.3 mm. The antenna insulator 118, 218 may extend proximally past the most proximal extent/end of the antenna elements 116a-i, 216a-i. This may reduce the risk of an electric short between the antenna elements and the outer conductor occurring. In other embodiments, the proximal end of the insulator may coincide with the proximal end of the antenna elements, or it may extend a shorter distance along the feed cable.

The ablation probe is arranged to emit radiation at an operating frequency. The operating frequency may be a microwave frequency. In some embodiments, the operating frequency of the ablation probe may be within a range of 10.0 GHz to 0.5 GHz. In some embodiments, the operating frequency of the ablation probe may be within a range of 1.0 to 4.0 GHz, or more preferably 2.4 GHz to 2.5 GHz. In particular, at a frequency of about 2.45 GHz. The antenna elements 116a-i, 216a-i extend in a direction along the length of the feed cable a length (L labelled in Figure 4) so that the antenna is tuned to the correct microwave frequency. The length L may be 25% of the wavelength of radiation at the operating frequency propagating in the antenna insulator material. For example, the length L may be in the range of 8 to 12 mm, preferably 9 to 11 mm, and more preferably about 10 mm.

The shape of the antenna elements 116, 216 shown in Figures 1 to 7b are to be understood as one example only. In other embodiments, the overlapping portions of the antenna elements may not extend in a direction parallel to the longitudinal axis of the ablation probe as shown, but may have other shapes or arrangements. For example, in some embodiments, the overlapping portions of the antenna elements may overlap each other, and may for example be woven or braided. In some embodiments, the overlapping portions of the antenna elements may extend helically around the longitudinal axis of the ablation probe.

Referring again to Figure 2 and 3a, the first antenna section 106a further comprise a heat-shrink sleeve 119 arranged to retain the overlapping portions 117b of the antenna elements 116a-i around the insulator. The heat-shrink sleave may form a tight-fitting layer of material over the antenna insulator 118 and antenna elements 116a-i to secure them in place. Although shown in combination with the embodiment of Figures 2 and 3a, the heat-shrink layer may be provided to retain the antenna elements of any embodiment described herein. The heat-shrink sleeve may extend proximally past the proximal end of the antenna elements along the feed cable so that the antenna elements are not exposed. This may reduce the risk of electrical shorts.

Other methods of holding the antenna elements 116a-i in place may be used. In yet other embodiments, the overlapping portions 117b of the antenna elements 116a-i may additionally or alternatively be embedded into the antenna insulator 118. This may be achieved by heating the antenna insulator to soften it, so that the overlapping portions 117b can be pushed into it, and then allowed to cool. In yet other embodiments, the overlapping portions 117b of the antenna elements 116a-i may be additionally or alternatively crimped around the insulator. In yet other embodiments, the first antenna section 106a may additionally or alternatively comprise one or more retaining bands arranged to secure the antenna elements 116a-i around the antenna insulator 118. The retaining bands may be radio-opaque marker bands, which may also add a visual reference to allow the user to determine the location of the antenna during use (e.g. using X-ray imaging). The radio-opaque marker bands may be made from any suitable material which is opaque to X-rays to act as a marker as is known in the art. The bands may be electrically conductive bands arranged to surround and retain the overlapping portions of the one or more antenna elements around the antenna insulator. The bands may be formed from a ring of solder applied around the antenna elements to hold them in place. Additionally or alternatively, the first antenna section may further comprise a wire or wires arranged to extend around the overlapping portions of the antenna elements to hold them in place. The wire(s) may be a polymer or metallic, and may be arranged to surround and retain the overlapping portions of the one or more antenna elements around the antenna insulator. Additionally or alternatively, the first antenna section may comprise a glue (e.g. an epoxy) arranged to retain the overlapping portions of the one or more antenna elements around the antenna insulator. For example, the glue may be used to secure the antenna elements to the antenna insulator material. Additionally or alternatively, the antenna insulator 118 may comprise one more recesses each arranged to receive a respective one of the antenna elements 116a-i. For example, one or more recessed portions may be machined or moulded into the surface of the insulator. The recesses may each be shaped to receive a respective one of the antenna elements (e.g. the overlapping portions of each element) so that the antenna element may lie in the recess and is held in position. In yet other embodiments the heat-shrink sleeve 119, embedding into the antenna insulator, retaining bands, wire(s) glue, recesses and/or crimping may be absent. The methods of retaining the antenna elements described in this paragraph may apply to any embodiment described herein.

The first antenna section 106a, 206a described above may be used in combination with a number of different types of second antenna section which is electrically coupled to the inner conductor. In the so far described embodiments, the second antenna section 106a, 206a comprises a straight monopole antenna formed by a portion of the inner conductor 108, 208. The second antenna section comprises a length of the inner conductor 108, 208 extending distally past the antenna elements 116a-i, 216a-i along the length of the ablation probe 100, 200. The second antenna section is thus formed from a part of the inner conductor extending distally from the feed point of the antenna i.e. distally from the most distal extent of the outer conductor 110, 210 along the length of the ablation probe.

In some embodiments, the second antenna section further comprises a dielectric resonator into which the inner conductor is inserted. Such an embodiment is illustrated in Figure 8, which shows an embodiment of the ablation probe 300 having an antenna 306 with corresponding components to other embodiments described herein, with the addition of a dielectric resonator 320. Any of the embodiments described herein may have such a dielectric resonator. The distal end of the inner conductor 308 extends into the dielectric resonator as can be seen in Figure 8. The dielectric resonator may be formed from a ceramic material. Different materials may be however used in other embodiments. The dielectric resonator may generally be formed from a rigid material.

In embodiments where the second antenna section comprises a dielectric resonator in which the inner conductor is inserted the antenna may further comprise a flexible hinge region 322. A proximal end of the dielectric resonator 320 is spaced apart from the distal end of the outer conductor 310 along the length of the coaxial cable to form the hinge region 322 between them. As can be seen in Figure 8, the proximal end of the dielectric resonator 320 is spaced apart from the antenna elements 316a-i along the length of the coaxial cable. The hinge portion is therefore formed from a section of the coaxial cable where the dielectric layer 312 of the cable is exposed. The hinge region 322 comprises a region of greater flexibility compared to the part of the antenna where the outer conductor is present and compared to the dielectric resonator 320. This provides a point of preferential bending of the ablation probe away from where components are connected together (e.g. where the dielectric resonator is connected to the inner conductor). This may help to avoid those connections from failing when the ablation probe is bent during use. The hinge region 322 may also protect the feed point 314 of the antenna 306 which is the centre of the ablation zone and the most critical part of the antenna by being exposed to the strongest electromagnetic field. The hinge region may, for example, extend a distance of 0.5 to 2 mm along the length of the ablation probe to allow a suitable degree of flexibility.

Although the hinge region is shown in use with the bent-back antenna elements of the present invention it may be used for other antenna designs. For example, more generally the hinge region may be provided between the dielectric resonator and outer conductor of any other antenna design.

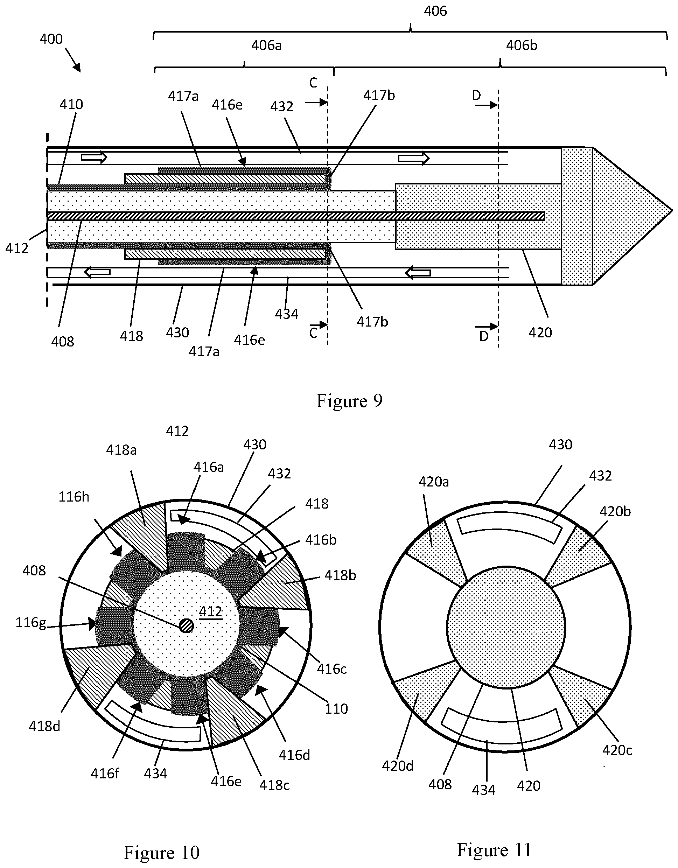

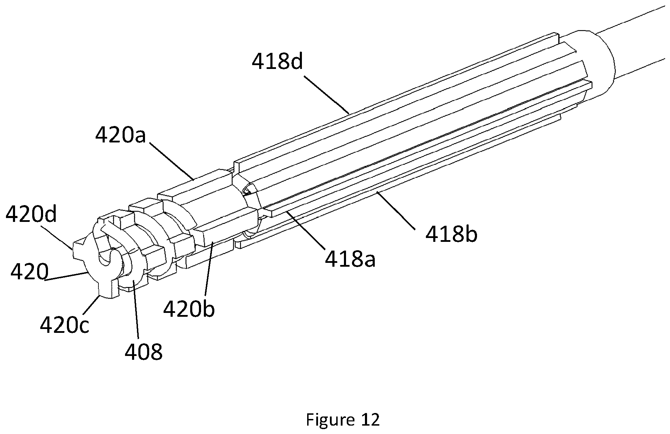

Another embodiment of an ablation probe 400 according to the present invention is illustrated in Figures 9, 10 and 11. Figure 9 shows a cross section through the distal end of the ablation probe 400 where the antenna 406 is located. Figures 10 and 11 show cross sections through planes C-C and D-D marked in Figure 8. Figure 12 shows the components of the antenna 406 of the ablation probe 400 without the catheter tube shown in Figures 9, 10 and 11.

The ablation probe 400 comprises features corresponding to those of the other embodiments disclosed herein, for which corresponding reference numerals have been used. The ablation probe 400 comprises a catheter tube 430 in which the components of the antenna and the coaxial cable are located. The catheter tube therefore may be considered to form part of the feeding cable of the ablation probe. Such a catheter tube may be provided for any other embodiment described herein.

The catheter tube 430 may be a tubular shaft such as a sheath or other such hollow elongate component. It may extend along the length of the ablation probe from its proximal end where it is connected to the handle to its distal end where the antenna is located. The catheter tube 430 may be formed from a material which has sufficient rigidity to allow the ablation probe to be inserted into tissue, but is sufficiently flexible to allow it to pass through the working channel of a delivery device. In other embodiments, the catheter tube may be more rigid to allow the ablation probe to be used percutaneously. The catheter tube may be formed from a super-elastic material. The catheter tube may be a single length of tube, or may comprise a number of separate lengths of tube in some embodiments.

In some embodiments, in order to help facilitate radiation of energy into target tissue and generate ablation the catheter tube 430 in the region of the antenna 406 may be made transparent to propagating microwaves. The section of the catheter tube 430 extending around the antenna 406, or immediately adjacent to the antenna, may therefore be free of any electrically conductive components. This helps to avoid the blocking of microwaves being emitted from the antenna 430. In some embodiments, the catheter tube may comprise one or more polymer materials. The polymer material or materials may be from the Fluoropolymer or Fluoroelastomer families, for example fluorinated ethylene propylene (FEP) and/or polytetrafluoroethylene (PTFE). In some embodiments, the catheter tube may comprise a polymer (e.g. PEEK) reinforced laminated tube. The entire length of the catheter tube may be made solely from a polymer material or materials, such as those mentioned in this paragraph or elsewhere herein (i.e. with no metallic or other electrically conducting material being present). These materials may allow the desired level of flexibility and durability of the catheter tube where it is stressed by high temperature, pressure, insertion and withdrawal forces when in use, while also being transparent to microwave radiation. In some embodiments, only the section of the catheter tube surrounding the antenna may be made solely from a polymer material(s) e.g. solely from a polymer material(s) from the Fluoropolymer or Fluoroelastomer families (e.g. fluorinated ethylene propylene (FEP) or polytetrafluoroethylene (PTFE)) or solely a polymer (e.g. PEEK) reinforced laminated tube. In such embodiments, other materials may be used in other parts of the catheter tube (e.g. away from the distal end) where no microwave radiation is present that would otherwise be impeded.

Referring to Figures 10 and 11, the outer surface of the dielectric resonator 420 of the second antenna section comprises a plurality of resonator protrusions 420a-420d. The resonator protrusions may be fins extending from the surface of the dielectric resonator and extending along the length of the dielectric resonator as shown in Figure 12. Four such protrusions are provided in the described embodiment, although other numbers may be provided in other embodiments. The resonator protrusions 420a-d may extend radially from an outer surface (e.g. a cylindrical outer surface) of the dielectric resonator 420. The resonator protrusions 420a-420d are arranged to space apart the interior wall of the catheter tube 430 (or another tube that surrounds it) from the surface of the dielectric resonator 420. The resonator protrusions 420a-d may be arranged to locate the catheter tube 430 concentrically with the dielectric resonator 420. For example, the resonator protrusions 420a-d may be spaced evenly around the circumference of the dielectric resonator and extend to equal distances from the longitudinal axis of the ablation probe to locate the catheter tube 430 concentrically. The resonator protrusions 420a-d may be formed integrally with the body of the dielectric resonator e.g. the shape of the dielectric resonator including the protrusions may be machined from a continuous piece of material. This may aid manufacture. In other embodiments, the protrusions may be separate components coupled to the surface of the dielectric resonator.

The first antenna section 406a of the ablation probe shown in Figures 9 to 12 comprises an antenna insulator 418 which comprises a plurality of insulator protrusions 418a-d. The insulator protrusions are formed by protrusions extending radially from the surface of the antenna insulator 418. The antenna protrusions may be fin shaped similarly to the resonator protrusions as shown in Figure 12. In the present embodiment, four insulator protrusions 418a-d are provided, but other numbers may be provided in other embodiments. The insulator protrusions 418a-d extend from an outer surface of the antenna insulator 418 around which the antenna elements 418a-h are arranged. In the present embodiment, the antenna protrusions 418a-d are located between adjacent antenna elements such that they extend between them (there are eight antenna elements in the present embodiment rather than nine of previously described embodiments to allow equal spacing of the antenna protrusions). The insulator protrusions 418a-d are arranged to locate the catheter tube 430 around the feed cable and around the first antenna section 406a. The insulator protrusions 418a-d may be arranged to locate the catheter tube 430 concentrically with the antenna insulator 418 and hence the other components of the first antenna section. The insulator protrusions 418a-d may be spaced evenly around the circumference of the antenna insulator 418 and extend to an equal radial distance from the longitudinal axis of the coaxial cable to locate the catheter tube 430 concentrically.

In embodiments having protrusions on the antenna insulator 418 the heat-shrink sleeve 119 may not be provided, with other means for holding the antenna elements in place as described elsewhere herein being used (e.g. one or more of embedding them into insulator material, adhesive or recesses in the surface of the insulator material).

Referring to the cross-section views shown in Figures 10 and 11, the antenna protrusions 218a-d and the resonator protrusions 220a-d are arranged to create spaces between the interior wall of the catheter tube 430 and the other components of the first antenna section or dielectric resonator respectively. In the present embodiment, this space is used for one or more coolant channels along which coolant can flow to cool the antenna. The flow of coolant may help control the temperature of the ablation probe during use. This may allow energy to be delivered to the surrounding tissue for an extended period of time without the ablation probe 100 overheating and being damaged, or causing injury to healthy tissue. The coolant may be a fluid, and may be water, saline solution, a cryogenic gas or any other suitable coolant known in the art.

The present embodiment comprises a first coolant conduit or tube 432 which extends along the length of the feeding cable. The first coolant conduit is located within the catheter tube 430 such that it extends between two of the antenna protrusions 418a, 18b and between two of the resonator protrusions 420a, 420b. The first coolant conduit 432 is arranged to carry a flow of coolant in a distal direction along the feed cable to (and through) the antenna 406. The first coolant conduit is therefore a coolant supply conduit. A similar second coolant conduit or tube 434 is provided to act as a coolant return conduit. The flow of coolant is shown by the arrows in Figure 9. The second coolant conduit 434 is arranged to extend along the length of the feeding cable between two of the antenna protrusions 418c, 418d and two of the resonator protrusions 420c, 420d. The distal end of the coolant conduits are fluidly coupled (e.g. by the cavity surrounding the dielectric resonator or additional coolant conduits or coolant carrying structure) such that a coolant circuit is formed. This allows coolant to be circulated to and from the antenna. For example, the proximal ends of the coolant conduits may be connected to a coolant pump or circulation device. By using the space between the resonator and antenna protrusions in this way a suitable location for the coolant conduits is provided while minimising the overall cross section of the ablation probe.

Although Figures 9, 10 and 11 show separate coolant conduits or tubes provided to carry coolant, in other embodiments these components may not be present i.e. coolant may flow along a coolant channel defined by the interior wall of the catheter tube and the surfaces of the resonator and/or antenna protrusions. Although two coolant channels are shown in the figures, there may be more in other embodiments so that a greater volume of coolant can be carried. In the presently described embodiment, the antenna protrusions and the resonator protrusions are aligned at the same angles around the longitudinal axis of the ablation probe. This may allow for straight, continuous coolant channels parallel to the longitudinal axis of the ablation probe to be provided in the gaps between the protrusions. This may reduce coolant flow back pressure. This may not be the case in other embodiments, with other arrangement of coolant channels possible. In some embodiments, only the antenna protrusions or only the dielectric protrusions may be provided through which coolant channels are located.

In any of the embodiments descried herein the antenna insulator may be arranged to allow coolant to flow through the respective first antenna section. This may help to mitigate back pressure in the coolant flow circuit. For example, coolant channels may be provided through the antenna insulator between the outer conductor of the coaxial cable and the overlapping portions of the antenna elements so that coolant can flow through the first antenna section.

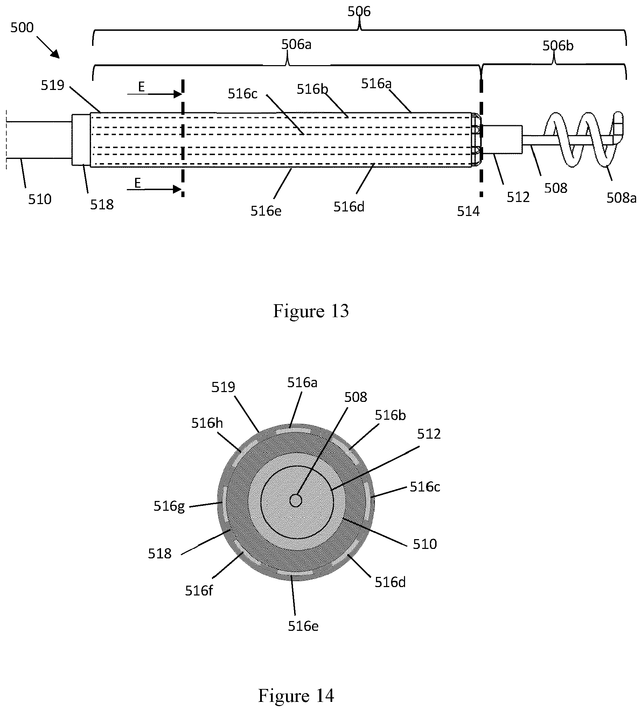

Figures 13 and 14 illustrate part of an ablation probe 500 according to another embodiment. In this embodiment, the antenna 506 comprises a different arrangement of second antenna section 506b compared to the other embodiments described herein. The embodiment shown in Figures 13 and 14 has components corresponding to those of previous embodiments described, with corresponding reference numerals used accordingly. The second antenna section 506b shown in Figure 13 comprises a helical portion 508a of the inner conductor 508. The helical portion extends from the most distal point of the straight section of the inner conductor, and forms a helix around the inner conductor extending proximally back along the length of the inner conductor. The second antenna section 506b may additionally comprise a dielectric resonator between the straight section of the inner conductor 508 and the helical portion 508a.

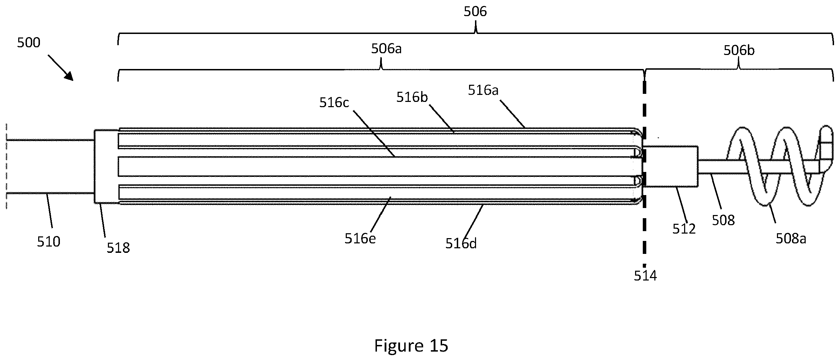

Figure 15 shows the same view as Figure 13, but without the heat-shrink 519 being present. This allows the antenna elements 516a-d to be more clearly visible extending along the length of the ablation probe, with gaps between them where the antenna insulator 518 beneath them is visible.

In the embodiments described above, the antenna insulator and catheter tube are separate components. In some embodiments, the catheter tube may comprise a multi-lumen extrusion (MLE). Part of the multi-lumen extrusion may be used to form the antenna insulator. The multi-lumen extrusion may be an extruded tube or structure having a plurality of lumens running along the length of the ablation probe, the lumens being defined by walls formed by the extruded material tube. The multi-lumen extrusion may be formed from an extruded polymeric material.

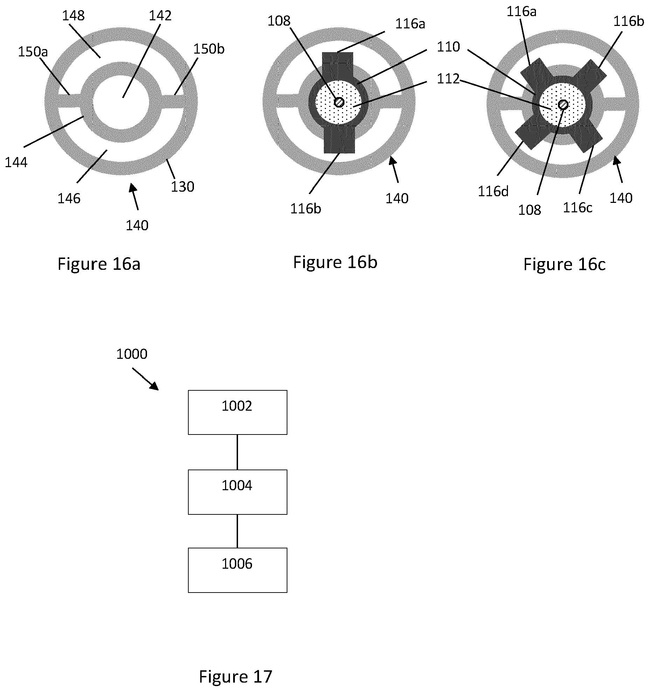

An example of a multi-lumen extrusion 140 which may form part of the ablation probe is illustrated in Figure 16a. Figure 16a shows the multi-lumen extrusion in isolation without the other components of the ablation probe for ease of explanation, and shows a cross section through the extrusion in a plane normal to the longitudinal axis of the ablation probe (e.g. equivalent to cross section A-A in Figure 2, or C-C in Figure 8). Figures 16b and 16c show the multi-lumen extrusion 140 of Figure 16a along with components of the first antenna section. Reference numbers corresponding to those of Figures 1 to 4 have been used in Figures 16a-c and it is to be understood that the multi-lumen extrusion 140 can be used with the embodiment shown in those figures or any other embodiment described herein.

The multi-lumen extrusion 140 comprises an outer extruded wall 130 which corresponds to the catheter tube of other embodiments described herein (e.g. catheter tube 430 shown in Figures 9 to 11). The multi-lumen extrusion 140 comprises an inner central lumen 142 which runs along the longitudinal axis of the ablation probe. The inner lumen 142 is defined by an inner extruded wall 144 of the multi-lumen extrusion 140. In the present embodiment, the inner extruded wall 144 forms a lumen having a generally circular cross section as shown in the figures. The coaxial cable may be located within the central lumen 142 as shown in Figures 16b and 16c. The multi-lumen extrusion 140 further comprises two outer lumens 146, 148 which in the present embodiment each partly surround the inner central lumen 142. Each outer lumen 146, 148 is defined by the outer surface of the inner extruded wall 144, the inner surface of the outer extruded wall 130 and two radial dividing walls 150a, 150b.

The antenna insulator 118 of the first antenna section 106a may be formed by part of the inner extruded wall 144 of the multi-lumen extrusion 140. As can be seen in Figures 16b and 16c, the part of the outer conductor 110 forming the antenna elements 116a, 116b, 116c, 116d may extend through or around the inner extruded wall 144 and then extend back proximally along the outer surface of the inner extruded wall 144. Part of the inner extruded wall 144 therefore extends between the overlapping portions of the antenna elements and the coaxial cable which they overlap to provide electric insulation between them. Figure 16b shows an example in which the first antenna section is has two antenna elements 116a, 116b and Figure 16c shows an example in which the first antenna section comprises four antenna elements 116a-d. In the embodiment shown in Figure 16b one of the antenna elements 116a, 116b is located in each of the outer lumens 146, 148. In the embodiment shown in Figure 16c two of the antenna elements 116a-d are located in each of the outer lumens 146, 148.

By forming the antenna insulator from part of a multi-lumen extrusion the number of components required to form the first antenna section can be reduced (e.g. a separate antenna component is not required). In some embodiments, the outer lumens 146, 148 may be arranged to carry a flow of coolant along the length of the ablation probe. For example, a first of the outer lumens 146 may carry a flow of coolant flowing in a distal direction along the length of the ablation probe with the second of the outer lumens 148 carrying a return flow of coolant in a proximal direction to form a coolant circuit as described elsewhere herein. In some embodiments, the lumens may contain a coolant flow tube or conduit in which the coolant flows (e.g. corresponding to the coolant conduits 432, 434 described in connection with Figures 9-11. The lumens may be fluidly coupled to a coolant pump as already described to circulate coolant.

Figures 16a-c show only one example of the arrangement of lumens that may be provided in the multi-lumen extrusion. In other embodiments, further outer lumens may be provided, for example there may be four outer lumens. In other examples, any other number of outer lumens may be provided (e.g. there may only be one). In yet other examples, further lumens may be provided in further layers around the outer-lumens shown in the figures.

Figure 17 illustrates a method of manufacturing an ablation probe. Specifically, manufacture of components of the antenna of an ablation probe. The ablation probe may be any of those described herein. The method 1000 comprises providing 1002 a coaxial cable comprising an inner conductor, an outer conductor and a dielectric therebetween. The method 1000 further comprises forming 1004 a first antenna section by shaping a part of the outer conductor of the coaxial cable such that it forms antenna elements. Each antenna element comprises an outwardly extending portion of the outer conductor extending in a radial direction away from a longitudinal axis of the feeding cable and an overlapping portion of the outer conductor extending proximally long the coaxial cable according to any of the embodiment described herein. The antenna elements may be formed by bending part of the outer conductor so that it has the desired shape i.e. bending it so that it extends outwards and back along the length of the coaxial cable.

The method 1000 further comprises providing an antenna insulator arranged to space apart the overlapping portions of the antenna elements from the part of the outer conductor which they overlap. The insulator may be inserted between the overlapping portions of the antenna elements and the outer conductor to provide electrical insulation between them according to any of the embodiments described herein. In other embodiments, the antenna insulator may be placed around the outer conductor before the antenna elements are shaped. This may allow the part of the outer conductor forming the antenna elements to be bent or shaped around the antenna insulator.

As described above, the outer conductor may in some embodiments comprise a plurality of braided conducting wire strands, the antenna elements each being formed by one or more of those strands. The step of forming 1004 the one or more antenna elements may therefore comprise: un-braiding the braided strands of part of the outer conductor of the coaxial cable; and shaping or bending the un-braided stands to form the one or more antenna elements of the first antenna section. As discussed above, the un-braided strands may be bent into the desired shape to extend away from and back along the coaxial cable. Each strand of the wire braid may be used to form each antenna element. That may not be the case however, and the strands may be partially re-braided to form each of the antenna elements. In some embodiments, the antenna elements may be overlapped or woven together to form a new braided section of the outer conductor as described above.

In embodiments where the outer conductor comprises a solid conducting sheath or tube of material around the dielectric, the step of forming 1004 the antenna elements of the first antenna section comprises: cutting the sheath or tube to form one or more strips integrally formed with the remainder of the outer conductor material from which they are cut; and shaping each of the one or more strips to form the one or more antenna elements of the first antenna section. The material of the outer conductor may be cut using any suitable method. Preferably laser cutting may be used, as this may provide accurate cutting. Laser cutting may also avoid damage to the dielectric of the cable by tuning the laser to a frequency suitable to cut only the metal of the outer conductor.

The method shown in Figure 17 is to be understood as providing steps to manufacture the first antenna section of the antenna for any if the ablation probes described herein. The method is to be understood as comprising further steps required to manufacture/assemble the other components of the ablation probe as would be apparent to the skilled person.

Various modifications will be apparent to the skilled person without departing form the scope of the claims. Any feature disclosed in connection with one embodiment may be used in combination with the features of another embodiment.

Although the appended claims are directed to particular combinations of features, it should be understood that the scope of the disclosure of the present invention also includes any novel feature or any novel combination of features disclosed herein either explicitly or implicitly or any generalisation thereof, whether or not it relates to the same invention as presently claimed in any claim and whether or not it mitigates any or all of the same technical problems as does the present invention.

Features which are described in the context of separate embodiments may also be provided in combination in a single embodiment. Conversely, various features which are, for brevity, described in the context of a single embodiment, may also be provided separately or in any suitable sub-combination. The applicant hereby gives notice that new claims may be formulated to such features and/or combinations of such features during the prosecution of the present application or of any further application derived therefrom.

For the sake of completeness, it is also stated that the term "comprising" does not exclude other elements or steps, the term "a" or "an" does not exclude a plurality, a single processor or other unit may fulfil the functions of several means recited in the claims and any reference signs in the claims shall not be construed as limiting the scope of the claims.

## Claims

1. A microwave ablation probe, comprising:
a dipole antenna arranged to emit microwave radiation to heat surrounding tissue; and
a feed cable arranged to supply electromagnetic energy to the dipole antenna, the feed cable comprising a coaxial cable having an inner conductor, an outer conductor and a dielectric therebetween, wherein:
the dipole antenna comprises a first antenna section forming a first pole of the dipole antenna and a second antenna section forming a second pole of the dipole antenna;
the first antenna section comprises one or more antenna elements each formed integrally with the outer conductor and comprising an outwardly extending portion extending away from a longitudinal axis of the feeding cable and an overlapping portion extending proximally along the length of the feeding cable; and
the first antenna section further comprises an antenna insulator between the overlapping portion of each of the one or more antenna elements and the part of the outer conductor which they overlap.

2. A microwave ablation probe according to claim 1, wherein:
the outer conductor is a braided outer conductor; and
the braided outer conductor comprises a plurality of braided wire strands, and wherein each antenna element is formed by one or more of the wire strands.

3. A microwave ablation probe according to claim 2, wherein the wire strands forming the antenna elements are overlapped or woven together such that the first antenna section is formed by an at least partly re-braided section of the outer conductor.

4. A microwave ablation probe according to claim 1, wherein the outer conductor comprises a solid conducing sheath or tube of material around the dielectric, and wherein the antenna elements of the first antenna section are each formed by part of the solid conducting sheath that is shaped to form the respective outwardly extending and overlapping portions of the antenna elements.

5. A microwave ablation probe according to any preceding claim, wherein the first antenna section comprises at least two antenna elements, the antenna elements being spaced apart circumferentially around the feeding cable, and preferably being evenly spaced around the feeding cable.

6. A microwave ablation probe according to any preceding claim, wherein the second antenna section comprises:
a length of the inner conductor extending distally past the antenna elements of the first antenna section along the length of the ablation probe; and
a dielectric resonator into which the inner conductor extends,
and optionally wherein a proximal end of the dielectric resonator is spaced apart from the antenna elements of the first antenna section along the length of the inner conductor to form a flexible hinge region therebetween.

7. A microwave ablation probe according to any preceding claim, further comprising a catheter tube in which the coaxial cable and the antenna are located, wherein optionally:
a) the region of the catheter tube which surrounds or is adjacent the antenna is formed from a polymer material or materials and is free of electrically conducting components, or
b) the entire length of the catheter tube is formed from a polymer material or materials and is free of electrically conducting components,
and further optionally wherein the polymer material(s) include:
i) a polymer or polymers from the Fluoropolymer or Fluoroelastomer families, for example fluorinated ethylene propylene (FEP) and/or polytetrafluoroethylene (PTFE); and/or
ii) a polymer (e.g. PEEK) reinforced laminated tube.

8. A microwave ablation probe according to claim 7, wherein:
the dielectric resonator comprises a plurality of radially extending resonator protrusions, the resonator protrusions extending from an outer surface of the dielectric resonator; and
optionally when dependent on claim 7 the catheter tube is spaced apart from the outer surface of the dielectric resonator by the plurality of resonator protrusions, and wherein further optionally the resonator protrusions are arranged to locate the catheter tube concentrically with the dielectric resonator.

9. A microwave ablation probe according to any preceding claim, wherein the first antenna section comprises a plurality of antenna elements and the antenna insulator comprises a plurality of radially extending insulator protrusions extending between the antenna elements, and optionally when dependent on claim 7 or claim 8 the insulator protrusions are arranged to locate the catheter tube concentrically with the antenna insulator.

10. A microwave ablation probe according to claim 8 or claim 9, further comprising one or more coolant channels between the resonator protrusions and/or the insulator protrusions.

11. A microwave ablation probe according to any preceding claim, wherein any one or more of:
a) the antenna insulator forms a flexible region of the ablation probe and comprises:
i) a solid flexible material; or
ii) a plurality of rigid beads,
c) the antenna insulator may allow flow of coolant therethrough;
d) the antenna insulator may comprise one or more airgaps; and/or
e) the feed cable comprises an extruded multi-lumen extrusion, and wherein the antenna insulator is formed by part of the multi-lumen extrusion.

12. A microwave ablation probe according to any preceding claim, wherein any one or more of:
a) the overlapping portions of the one or more antenna elements are embedded into the antenna insulator;
b) the overlapping portions of the one or more antenna elements are crimped around the antenna insulator;
c) the first antenna section further comprise a heat-shrink sleeve arranged to surround and retain the overlapping portions of the one or more antenna elements around the antenna insulator;
d) the first antenna section comprises a wire arranged to surround and retain the overlapping portions of the one or more antenna elements around the antenna insulator;
e) the first antenna section further comprises one or more radio-opaque marker bands arranged to surround and retain the overlapping portions of the one or more antenna elements around the antenna insulator;
f) the first antenna section comprises a glue arranged to retain the overlapping portions of the one or more antenna elements around the antenna insulator;
g) the first antenna section further comprises one or more electrically conductive bands arranged to surround and retain the overlapping portions of the one or more antenna elements around the antenna insulator, wherein optionally the bands are formed from a ring of solder around the antenna elements; and/or
h) the antenna insulator of the first antenna section may comprise one more recesses each arranged to receive a respective one of the antenna elements.

13. A method of manufacturing an ablation probe, the ablation probe comprising:
a dipole antenna arranged to emit microwave radiation to heat surrounding tissue, the dipole antenna comprising a first antenna section forming a first pole of the dipole antenna and a second antenna section forming a second pole of the dipole antenna; and
a feed cable comprising a coaxial cable arranged to supply electromagnetic energy to the dipole antenna, the method comprising:
providing a coaxial cable comprising an inner conductor, an outer conductor and a dielectric therebetween;
forming the first antenna section by shaping a part of outer conductor such that it forms one or more antenna elements each comprising an outwardly extending portion of the outer conductor extending away from a longitudinal axis of the feeding cable and an overlapping portion of the outer conductor extending proximally along the length of coaxial cable; and
providing an insulator between the overlapping portions of the one or more antenna elements and the part of the outer conductor which they overlap.

14. A method of manufacturing an ablation probe according to claim 13, wherein the outer conductor comprises a plurality of braided conducting wire strands, and forming the antenna elements of the first antenna section comprises:
un-braiding the braided strands of part of the outer conductor of the coaxial cable; and
shaping the un-braided stands to form the one or more antenna elements of the first antenna section.

15. A method of manufacturing an ablation probe according to claim 13, wherein the outer conductor comprises a solid conducting sheath or tube of material around the dielectric, and forming the antenna elements of the first antenna section comprises:
cutting the sheath or tube to form one or more strips integrally formed with the remainder of the outer conductor material from which they are cut; and
shaping each of the one or more strips to form the one or more antenna elements of the first antenna section.
